# DEMANDE DE BREVET EUROPEEN

(11) **EP 1 506 780 A2**
(43) Date de publication de la demande: **16.02.2005**
(21) Numéro de dépôt: 04025511.9
(22) Date de dépôt: 30.03.2000
(51) Int. Cl.: A61K 31/70, A61K 9/00

(54) **Formulations pharmaceutiques de macrolides seuls ou associés à d'autres principes actifs**

(30) Priorité: 30.03.1999 FR 9903978
(62) Demande divisionnaire de: 00915252.1
(71) Demandeur: CLL PHARMA, 06200 Nice (FR)
(72) Inventeur: Zakarian, Noel, 13009 Marseille (FR); Gimet, René, 06560 Valbonne (FR); Laruelle, Claude, 06270 Villeneuve-Loubet (FR); Toselli, Dominique, 06000 Nice (FR)
(74) Mandataire: Goulard, Sophie

(57) **Abrégé**

La présente invention concerne des comprimés dispersible renfermant des macrolides en tant que principes actifs, seuls ou associés à d'autres principes actifs, ainsi qu'à leur procédé de préparation. Ces comprimés dispersibles sont caractérisés en ce que le macrolide est choisi dans le groupe constitué par la roxithromycine à raison de 15 % en poids du poids total desdits compriméset la spiramycine à raison de 19 % en poids du poids total desdits comprimés, et est présent sous forme de base, et en ce qu'ils comprennent au moins un désintégrant, dans des proportions comprises entre 1% et 25% du poids total desdits comprimés, et au moins un édulcorant.

Application : antibiothérapie

## Description

La présente invention concerne des comprimés dispersibles renfermant des macrolides en tant que principe actifs, seuls ou associés à d'autres principes actifs, ainsi qu'à leur procédé de préparation.

En thérapeutique, la simplicité d'utilisation des comprimés, a toujours été considérée comme un avantage majeur, notamment dans le cadre de traitements ambulatoires, comme en témoigne le très grand nombre de spécialités pharmaceutiques qui se présentent sous cette forme.

Toutefois, certains patients et, notamment, les enfants et les personnes âgées, connaissent des difficultés de déglutition telles qu'il leur est difficile et, par conséquent, désagréable d'ingérer des comprimés, même avec une prise associée de liquide.

C'est la raison pour laquelle il est souhaitable de disposer de comprimés aptes à se désintégrer dans un faible volume de liquide, de manière à pouvoir être ingérés sous la forme de solutions ou de suspensions buvables.

Or, de nombreux principes actifs sont connus pour présenter une amertume très difficile à masquer, lorsqu'ils se présentent sous la forme de solutions, ou de suspensions buvables.

C'est le cas en particulier des macrolides, qu'ils soient utilisés seuls ou en association avec d'autres principes actifs. Les macrolides ont en commun de comprendre un noyau lactonique central constitué de 14 à 16 chaînons avec peu ou pas de doubles liaisons et pas d'azote. Un ou plusieurs sucres aminés et/ou neutres (désosamine, cladinose, mycarose, mycaminose) sont fixés par des liaisons α ou β-glycosidiques sur ce noyau, encore appelé aglycone. On peut citer comme macrolides dérivés naturels, l'érythromycine A à F, l'oléandomycine, la spiramycine, la midécamycine et la troléandomicyne et comme macrolides semi-synthétiques, la roxithromycine, la dirithromycine, la clarithromycine, la flurithromycine et la rokitamycine. Les azolides à 15 chaînons, qui possèdent un atome d'azote endocyclique, comme l'azithromycine font également partie des macrolides (A. Bryskier, 1995 dans *Le bon usage des macrolides*, page 8, Classification des macrolides, Ed. Phase 5).

Toutefois, cette amertume est plus ou moins marquée suivant les caractéristiques physico-chimiques des macrolides. Par exemple, la troléandomycine est pratiquement dénuée d'amertume (*Traité de chimie thérapeutique*, 1992, vol. 2, Médicaments Antibiotiques TEC & DOC Lavoisier, Editions Médicales Internationales), tandis que la pristinamycine, l'azithromycine, la roxithromycine, la clarithromycine et la spiramycine ont une amertume très prononcée.

Aussi de nombreuses techniques ont-elles été proposées pour masquer l'amertume de ces principes actifs et, en particulier celui de la roxithromycine, de la clarithromycine et de la spiramycine.

D'une manière générale, ces techniques consistent soit à procéder à un enrobage plus ou moins complexe du principe actif (Demande de Brevet Français n° 2 669 533 ; Demande Internationale WO 97/16174), soit plus simplement à tenter de masquer le goût par l'emploi d'un édulcorant adapté, le plus souvent associé à une quantité importante de saccharose (Demande de Brevet Français n° 2 696 346 ; spécialité pharmaceutique Rulid® 50 mg, poudre pour suspension buvable).

Ainsi la Demande de Brevet Français n° 2 669 533 décrit un procédé de fabrication de granulés dispersibles renfermant de la spiramycine et destinés à masquer le goût de ce principe actif. Dans ce procédé, la spiramycine est encapsulée par de l'albumine par une technique qui nécessite la mise en oeuvre de solvants organiques tels que l'isooctane et leur élimination en fin de procédé, puis les capsules ainsi obtenues sont diluées par un mélange de sucres (lactose + fructose). La technique d'encapsulation de la spiromycine, bien que performante, est très coûteuse car elle ne permet que la fabrication de quantités réduites de composition pharmaceutique et exige des étapes de recyclage de solvants longues et onéreuses.

C'est la raison pour laquelle la Demande Internationale n° WO 97/16174 propose, elle, un procédé permettant de préparer des granulés dispersibles d'un macrolide et, notamment, de clarithromycine, sans utiliser de solvants organiques. Dans ce procédé, le macrolide est soumis à une granulation après mélange avec un polymère d'acide acrylique ramifié à haut pouvoir de réticulation. Cette granulation est réalisée en présence d'eau et est, éventuellement, suivie d'une seconde granulation qui, elle, est effectuée en présence d'une solution aqueuse d'un agent liant comme la polyvinylpyrrolidone.

La Demande de Brevet Français n° 2 696 346 propose également de préparer des formulations de spiramycine au goût amélioré et se présentant sous la forme de granulés à dissoudre ou à disperser dans de l'eau. Ces formulations contiennent un édulcorant particulier, à savoir l'acésulfame de potassium, et du saccharose en forte proportion - puisque le rapport pondéral spiramycine/saccharose est compris entre 1/1 et 1/9 - afin de masquer l'amertume de la spiramycine.

Toutefois ces dernières formulations, tout comme les formulations obtenues par enrobage comme proposées dans FR-A-2 669 533 et WO-A-97/16174, présentent certains inconvénients et, notamment, celui de ne pas suffisamment masquer l'amertume des macrolides qu'elles renferment. De plus, les quantités de sucre(s) présentes dans les formulations décrites dans FR-A-2 669 533 et FR-A-2 696 346 rendent l'administration de ces formulations contre-indiquée aux patients diabétiques.

Récemment, un comprimé dispersible, sans enrobage et exempt de sucre, a bien été proposée pour la josamycine (JOSACINE® dispersible), mais cette dernière est connue pour avoir un goût beaucoup moins amer que les autres macrolides et pour ne présenter aucune difficulté technique pour la formulation. Dans ce comprimé, la josamycine est présente sous forme de propionate, dans une quantité correspondant à 50 % du poids dudit comprimé.

Toutefois, à ce jour, aucun comprimé dispersible, exempt de sucre et présentant un goût convenable, n'a jamais été proposé pour les macrolides les plus amers, comme la spiramycine et la roxithromycine. En conséquence, les Inventeurs se sont donnés pour but de pallier ce manque et de développer des comprimés dispersibles renfermant des principes actifs et, notamment des macrolides très amers, et qui, bien qu'étant exempt de sucres, conduisent, lorsqu'ils se désintègrent dans de l'eau, à des suspensions buvables ayant un goût tout à fait acceptable de manière à ce que ces suspensions ne soient pas désagréables à avaler.

La présente invention a, donc, pour objet des comprimés dispersibles qui contiennent un macrolide en tant que principe actif, seul ou en association avec un autre principe actif, lesquels comprimés sont caractérisés en ce que le macrolide n'est pas enrobé, est choisi dans le groupe constitué par la roxithromycine en une proportion de 15% en poids du poids total des comprimés et la spiramycine en une proportion de 19% en poids du poids total des comprimés, et est présent sous forme de base, et en ce qu'ils comprennent au moins un désintégrant, dans des proportions comprises entre 1 % et 25% de poids total, et au moins un édulcorant.

Au sens de la présente invention, on entend par comprimés "dispersibles", des comprimés capables de se désintégrer complètement en moins de 3 minutes lorsqu'ils sont mis dans un liquide comme de l'eau, et de conduire, ainsi, à une suspension buvable dont l'homogénéité peut être aisément obtenue en agitant celle-ci, par exemple, au moyen d'une petite cuillère. De tels comprimés peuvent, toutefois, être également avalés directement avec une quantité de liquide propre à faciliter leur déglutition.

Malgré l'absence de sucres et, notamment, de saccharose, les comprimés conformes à l'invention présentent de manière surprenante un goût nettement plus agréable que celui présenté par les poudres et granulés dispersibles proposés jusqu'à présent pour les macrolides amers, et ce, même lorsqu'ils renferment des quantités de macrolides élevées.

Le désintégrant est l'agent qui permet aux comprimés de se désintégrer complètement en présence d'un liquide et ce, en un temps relativement court, puisqu'inférieur à 3 minutes, et au(x) principe(s) actif(s) d'être libérés dans ce liquide ; son choix est, donc, particulièrement important.

Aussi, selon une autre disposition avantageuse de l'invention, le désintégrant est choisi dans le groupe constitué par la polyvinylpyrrolidone, le croscarmellose sodique et leurs mélanges.

Selon une disposition particulièrement préférée de l'invention, on utilise de la polyvinylpyrrolidone dans des proportions comprises entre 1% et 16% du poids total des comprimés, ou le croscarmellose sodique dans des proportions comprises entre 1% et 15% du poids total des comprimés ou encore le mélange des deux dans un rapport compris entre 1:1 et 4:1.

Selon encore une disposition avantageuse de l'invention, l'édulcorant est choisi dans le groupe constitué par l'aspartame, la saccharine sodique, l'acésulfame de potassium, le glycérinate d'ammonium et leurs mélanges.

Selon un mode de réalisation préféré de l'invention, on utilise un mélange de deux édulcorants dans un rapport compris entre 1:1 et 2:1, ledit mélange représentant en poids entre 1 et 20% du poids total des comprimés.

Selon un autre mode de réalisation préféré de l'invention, le macrolide est associé à un dérivé nitro-imidazolé. A titre d'exemples de tels dérivés, on peut citer le métronidazole, le tinidazole ou encore l'ornidazole.

Dans ce cas, le macrolide est, de préférence, de la spiramycine, tandis que le dérivé nitro-imidazolé est de préférence, du métronidazole.

Outre un macrolide, un désintégrant et un édulcorant, les comprimés selon l'invention contiennent d'autres excipients, dans des proportions qui sont choisies en fonction des propriétés physico-chimiques du macrolide qu'ils renferment.

Ces excipients sont sélectionnés dans le groupe constitué par les agents diluants, les agents tensioactifs, les agents lubrifiants et les agents d'écoulement.

Les comprimés contiennent, en outre, au moins un arôme qui contribuent à leur donner un goût acceptable pour les patients.

Les agents diluants facilitent les opérations de compression nécessaires à l'obtention de comprimés et donnent une dureté adéquate à ces derniers.

Selon l'invention, le ou les agents diluants peuvent notamment être choisis dans le groupe constitué par la cellulose microcristalline, le lactose, l'hydroxypropylméthylcellulose (HPC) et l'amidon prégélatinisé. De préférence on utilise la cellulose microcristalline dans des proportions comprises entre 5% et 50% du poids total des comprimés.

Les comprimés selon l'invention contiennent également un ou plusieurs agents tensioactifs, par exemple des polysorbates ou du laurylsulfate de sodium, dans des proportions comprises entre 0,1% et 3% de leur poids total.

Ils contiennent aussi un ou plusieurs agents lubrifiants tels que le stéarate de magnésium et le stéarate de calcium. Ces agents lubrifiants, dont le rôle est de réduire les frictions pendant les opération de compression, sont avantageusement présents dans des proportions comprises entre 0,5% et 3% du poids total des comprimés.

Parmi les agents d'écoulement susceptibles d'être inclus dans les comprimés selon l'invention, on peut citer notamment la silice colloïdale, le talc, l'acide stéarique et le stéarate de magnésium ; ces agents d'écoulement, qui évitent que les composants des comprimés ne forment des agrégats au cours de la préparation de ces comprimés et qui réduisent également les frictions pendant les opération de compression, sont présents dans des proportions comprises entre 0,1% et 3% du poids total des comprimés.

Le ou les arômes sont choisis en fonction de l'âge des patients (adultes ou enfants) auxquels les comprimés sont destinés et sont présents dans des proportions comprises entre 0,5% et 15% du poids total de ces comprimés.

Parmi les arômes susceptibles d'être utilisés, on peut citer les arômes menthe, chocolat, caramel, vanille, fraise, réglisse et leurs mélanges.

Les arômes menthe et vanille/caramel sont particulièrement préférés. L'arôme menthe est généralement présent dans des proportions comprises entre 1% et 7% poids total des comprimés, tandis que l'arôme vanille/caramel est, lui, présent dans des proportions comprises entre 1% et 10% du poids total desdits comprimés.

Les comprimés dispersibles selon l'invention offrent les avantages suivants :
- facilité d'emploi en traitement ambulatoire,
- précision du dosage unitaire,
- facilité de dispersion dans un liquide,
- goût agréable,
- facilité de déglutition en cas d'ingestion directe, c'est-à-dire sans dispersion préalable dans un liquide,
- absence de sucres et, notamment, de saccharose, les rendant particulièrement adaptés au traitement de patients diabétiques.

L'invention a, aussi, pour objet un procédé de préparation de comprimés dispersibles tels que précédemment définis, lequel procédé est caractérisé en ce qu'il comprend :
- le mélange du ou des principes actifs avec 30% à 60% de la quantité de désintégrant(s) destinée à être présente dans les comprimés,
- la granulation humide du mélange résultant en présence d'un liquide de mouillage contenant de l'eau et au moins un agent tensioactif,
- le séchage des granulés ainsi obtenus,
- l'ajout à sec des 40 à 70 % restant du ou des désintégrants, de l'édulcorant ou des édulcorants, du ou des agents diluants, agents lubrifiants, agents d'écoulement et du ou des arômes, et,
- la compression du mélange résultant.

L'invention sera mieux comprise au moyen du complément de description qui suit et qui se réfère à des exemples de réalisation de comprimés dispersible conformes à l'invention. Il va de soi, toutefois, que ces exemples sont donnés uniquement à titre d'illustrations de l'invention et n'en constituent nullement une limitation.

### Exemple 1 :

### Comprimés dispersibles contenant de la spiramycine base 0,75 MUI* en association avec du métronidazole ; arôme menthe

Des comprimés dispersibles pesant 1000 mg chacun et contenant 0,75 MUI de spiramycine base sont préparés avec les ingrédients suivants :

| | | |
|---|---|---|
| Spiramycine base | 190 mg (0,75 MUI)** | 19% |
| Métronidazole | 125 mg | 12,5% |
| Polyvinylpyrrolidone | 22,5 mg | 2,25% |
| Croscarmellose sodique | 75 mg | 7,5% |
| Polysorbate | 3,8 mg | 0,38% |
| Cellulose microcristalline. | 448,7 mg*** | 44,87% |
| Aspartame | 60 mg | 6% |
| Saccharine sodique | 30 mg | 3,00% |
| Arôme menthe | 30 mg | 3,00% |
| Silice Colloïdale | 5 mg | 0,5% |
| Stéarate de magnésium | 10 mg | 1,00% |

| | | |
|---|---|---|
| * MUI correspond à des Millions d'Unités Internationales et traduit l'activité d'un antibiotique ; elle est mesurée par comparaison de l'inhibition de la croissance de microorganismes sensibles induite par des concentrations connues de l'antibiotique à tester et d'une substance de référence (Pharmacopée 1997). ** la quantité de spiramycine est ajustée en fonction de son titre. | | |
| *** la quantité de cellulose microcristalline est ajustée en fonction de la quantité de spiramycine pour obtenir une masse finale de 1000 mg. | | |

### Exemple 2 : Comprimés dispersibles contenant de la roxithromycine base ; arôme menthe

Des comprimés dispersibles pesant 1000 mg chacun et contenant 150 mg de roxithromycine base sont préparés avec les ingrédients suivants :

| | | |
|---|---|---|
| Roxithromycine base | 150 mg | 15% |
| Crospovidone | 22,5 mg | 2,25% |
| Croscarmellose sodique. | 62,5 mg | 6,25% |
| Polysorbate | 3,8 mg | 0,38% |
| Cellulose microcristalline66,62% | 666,2 mg | |
| Aspartame | . 40 mg | 4,00% |
| Saccharine sodique | 20 mg | 2,00% |
| Arôme menthe | 20 mg | 2,00% |
| Silice colloïdale. | 5 mg | 0,5% |
| Stéarate de magnésium | 10 mg | 1,00% |

Tous les comprimés préparés conformément aux exemples 1 et 2 se sont révélés aptes à se désintégrer complètement en moins de 3 minutes, une fois mis dans un verre d'eau. Par ailleurs, des tests visant à comparer le goût des suspensions obtenues par dispersion des comprimés préparés conformément aux exemples 1 et 2 par rapport à celui de suspensions obtenues par dispersion des formes galéniques dispersibles actuellement disponibles pour les mêmes macrolides (Rovamycine® granulés pour la spiramycine, et Rulid® 50 mg, poudre pour suspension buvable pour la roxithromycine) ont montré que les comprimés selon l'invention permettent de mieux masquer l'amertume des macrolides et conduisent à des suspensions buvables dont le goût est notablement plus agréable.

## Revendications

1. Comprimé dispersible contenant un macrolide en tant que principe actif, seul ou en association avec un autre principe actif, **caractérisé en ce que** le macrolide n'est pas enrobé, est choisi dans le groupe constitué par la roxithromycine en une proportion de 15% de leur poids total, et la spiramycine en une proportion de 19% en poids de leur poids total, et est présent sous forme de base, et en qu'il comprend au moins un désintégrant, dans des proportions comprises entre 1% et 25% du poids total dudit comprimé, et au moins un édulcorant.

2. Comprimé dispersible selon la revendication 1, **caractérisé en ce que** le désintégrant est choisi dans le groupe constitué par la polyvinylpyrrolidone, le croscarmellose sodique et leurs mélanges.

3. Comprimé dispersible selon la revendication 2, **caractérisé en ce qu'**il contient de la polyvinylpyrrolidone dans des proportions comprises entre 1% et 16% du poids total dudit comprimé, ou du croscarmellose sodique dans des proportions comprises entre 1 % et 15% du poids total dudit comprimé, ou un mélange des deux dans un rapport compris entre 1:1 et 4:1.

4. Comprimé dispersible selon l'une quelconque des revendications 1 à 3, **caractérisé en ce que** l'édulcorant est choisi dans le groupe constitué par l'aspartame, la saccharine sodique, l'acésulfame de potassium, le glycérinate d'ammonium et leurs mélanges.

5. Comprimé dispersible selon la revendication 4, **caractérisé en ce qu'**il contient le mélange de deux édulcorants dans un rapport compris entre 1:1 et 2:1, ledit mélange représentant en poids entre 1 et 20% du poids total dudit comprimé.

6. Comprimé dispersible selon l'une quelconque des revendications 1 à 5, **caractérisé en ce que** le macrolide est associé avec un dérivé nitro-imidazolé.

7. Comprimé dispersible selon la revendication 6, **caractérisées en ce que** le macrolide est la spiramycine et le dérivé nitro-imidazolé est le métronidazole.

8. Comprimé dispersible selon l'une quelconque des revendications 1 à 7, **caractérisé en ce qu'**il contient de plus au moins un excipient choisi dans le groupe constitué par les agents diluants, les agents tensioactifs, les agents lubrifiants, les agents d'écoulement, et un ou plusieurs arômes.

9. Comprimé dispersible selon la revendication 8, **caractérisé en ce qu'**il contient au moins un agent diluant choisi dans le groupe constitué par la cellulose microcristalline, le lactose, l'hydroxypropylméthyl-cellulose et l'amidon prégélatinisé.

10. Comprimé dispersible selon la revendication 9, **caractérisé en ce que** la cellulose microcristalline est présente dans des proportions comprises entre 5% et 50% du poids total dudit comprimé.

11. Comprimé dispersible selon la revendication 8, **caractérisé en ce qu'**il contient au moins un agent tensioactif choisi dans le groupe constitué par les polysorbates et le laurylsulfate de sodium, dans des proportions comprises entre 0,1% et 3% du poids total dudit comprimé.

12. Comprimé dispersible selon la revendication 8, **caractérisé en ce qu'**il contient du stéarate de magnésium en tant qu'agent lubrifiant, dans des proportions comprises entre 0,5% et 3% du poids total dudit comprimé, et un agent d'écoulement dans des proportions comprises entre 0,1% et 3% du poids total dudit comprimé.

13. Comprimé dispersible selon la revendication 12, **caractérisé en ce qu'**il contient de la silice colloïdale en tant qu'agent d'écoulement.

14. Comprimé dispersible selon la revendication 8, **caractérisé en ce qu'**il contient au moins un arôme choisi dans le groupe constitué par les arômes menthe, chocolat, caramel, vanille, fraise, réglisse et leurs mélanges, dans des proportions comprises entre 0,5% et 15% du poids total dudit comprimé.

15. Comprimé dispersible selon la revendication 14, **caractérisé en ce que** l'arôme menthe est présent dans des proportions comprises entre 1% et 7% du poids total dudit comprimé, tandis que l'arôme vanille/caramel est présent dans des proportions comprises entre 1% et 10% du poids total dudit comprimé.

16. Procédé de préparation d'un comprimé dispersible selon l'une quelconque des revendications 1 à 15, **caractérisé en ce qu'**il comprend :
- le mélange du ou des principes actifs avec 30% à 60% de la quantité de désintégrant(s) destinée à être présente dans ledit comprimé,
- la granulation humide du mélange résultant en présence d'un liquide de mouillage contenant de l'eau et au moins un agent tensioactif,
- le séchage des granulés ainsi obtenus,
- l'ajout à sec des 40 à 70 % restant du ou des désintégrants, de l'édulcorant ou des édulcorants, du ou des agents diluants, agents lubrifiants, agents d'écoulement et du ou des arômes, et
- la compression du mélange résultant.
